(19) Europäisches Patentamt
European Patent Office
Office européen des brevets

(11) Veröffentlichungsnummer: **0 372 537 A2**

(12) **EUROPÄISCHE PATENTANMELDUNG**

(21) Anmeldenummer: **89122520.3**

(22) Anmeldetag: **06.12.89**

(51) Int. Cl.5: **C07K 5/02, C07K 5/06, A61K 37/64**

Patentansprüche für folgende Vertragsstaaten: ES + GR.

(30) Priorität: **09.12.88 DE 3841520**

(43) Veröffentlichungstag der Anmeldung: **13.06.90 Patentblatt 90/24**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim(Taunus)(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim(Taunus)(DE)**

(54) **Enzym-hemmende Harnstoffderivate von Dipeptiden, Verfahren zu ihrer Herstellung, diese enthaltende Mittel und ihre Verwendung.**

(57) Die vorliegende Erfindung betrifft Verbindungen der Formel I

$$R^1-\overset{R^5}{\overset{|}{N}}-\overset{O}{\overset{\|}{C}}-A-B-NH-\overset{R^2}{\overset{|}{CH}}-\overset{OH}{\overset{|}{CH}}-\overset{R^3}{\overset{|}{CH}}-(CH_2)_p-X-(CH_2)_q-R^4 \qquad (I)$$

in welcher
A und B unabhängig voneinander eine Aminosäure bedeuten,
X wahlweise abwesend sein kann oder für -O-, -S-, $-CF_2-$, -CO- oder $-CHR^8$ steht,
p und q unabhängig voneinander 0, 1, 2, 3 oder 4 bedeuten
und $R^1$ bis $R^4$ in der Beschreibung definiert sind, sowie deren Salze.

Die Erfindung betrifft ferner ein Verfahren zur Herstellung der Verbindungen der Formel I sowie deren Verwendung als Heilmittel.

EP 0 372 537 A2

## Enzym-hemmende Harnstoffderivate von Dipeptiden, Verfahren zu ihrer Herstellung, diese enthaltende Mittel und ihre Verwendung

Aus der EP-A-255 082 sind Di- und Tripeptidderivate bekannt, die am N-Terminus u.a. ein monosubstituiertes Harnstofffragment tragen können und eine Renin-inhibitorische Wirkung aufweisen.

EP-A-283 970 beschreibt Harnstoffderivate von Dipeptiden mit Renin-inhibitorischer Wirkung.

Es wurde nun überraschend gefunden, daß disubstituierte Harnstoffderivate von Dipeptiden, die sich von den bekannten Verbindungen der EP-A-255 082 durch einen zusätzlichen Substituenten am N-terminalen Stickstoffatom und von den in EP-A-283 970 beschriebenen Verbindungen durch die Natur des Substituenten $R^4$ unterscheiden, in vitro und in vivo das Enzym Renin und retrovirale Aspartylproteasen hochwirksam hemmen.

Die vorliegende Erfindung betrifft Verbindungen der Formel I

$$R^1-\overset{\overset{R^5}{|}}{N}-\overset{\overset{O}{\|}}{C}-A-B-NH-\overset{\overset{R^2}{|}}{CH}-\overset{\overset{OH}{|}}{CH}-\overset{\overset{R^3}{|}}{CH}-(CH_2)_p-X-(CH_2)_q-R^4 \qquad (I)$$

in welcher

$R^1$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,

($C_3$-$C_8$)-Cycloalkyl, ($C_3$-$C_8$)-Cycloalkyl-($C_1$-$C_6$)-alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,

($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_3$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet,

A einen N-terminal über -$NR^6$ mit -CO- und C-terminal über -CO- mit B verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin (Z)-Dehydrophenylalanin, (E)-Dehydrophenylalanin, (1,3-Dioxolan-2-yl)alanin, N-Pyrrolylalanin, (1-, 3-oder 4-Pyrazolyl)alanin, 4-(Thiazolyl)alanin, (2-, 4- oder 5-Pyrimidyl)alanin, Cyclopentylglycin, tert.Butylglycin, Phenylserin und $R^6$ Wasserstoff, ($C_1$-$C_6$)-Alkyl, Formyl, ($C_1$-$C_6$)-Alkoxycarbonyl oder Benzyloxycarbonyl bedeutet,

B einen N-terminal über -$NR^7$- mit A und C-terminal über -CO- mit

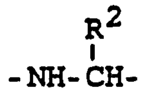

verknüpften Rest einer natürlichen oder unnatürlichen Aminosäure, wie für A definiert, und $R^7$ Wasserstoff, $(C_1-C_6)$-Alkyl, Formyl, $(C_1-C_6)$-Alkoxycarbonyl oder Benzyloxycarbonyl bedeutet,
$R^2$ Wasserstoff, $(C_1-C_{10})$-Alkyl, $(C_4-C_7)$-Cycloalkyl, $(C_4-C_7)$-Cycloalkyl-$(C_1-C_6)$-alkyl, $(C_6-C_{14})$-Aryl, $(C_6-C_{14})$-Aryl-$(C_1-C_6)$-alkyl oder (Heterocyclyl)-$(C_1-C_6)$alkyl bedeutet, wobei der Heterocyclus 4-7 Ringglieder, davon 1 oder 2 Schwefel-und/oder Sauerstoffatome, besitzt,
$R^3$ Wasserstoff, $(C_1-C_{10})$-Alkyl, $(C_6-C_{14})$-Aryl oder $(C_6-C_{14})$-Aryl-$(C_1-C_6)$-alkyl bedeutet,
X wahlweise abwesend sein kann oder für -O-, -S-, $-CF_2-$, -CO- oder $-CHR^8-$ steht, worin
$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,
$R^4$ -OH, $-NH_2$ oder Heteroaryl, das auch teilweise oder vollständig hydriert sein kann, und das gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet,
p und q unabhängig voneinander 0, 1, 2, 3 oder 4 bedeuten,
$R^5$ die gleiche Bedeutung wie $R^1$ besitzt oder aber zusammen mit $R^1$ und dem sie verbindenden Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet,
sowie deren physiologisch verträgliche Salze.

Die Chiralitätszentren in den Verbindungen der Formel I können die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aralkyl. Unter Aralkyl versteht man einen mit $(C_1-C_6)$-Alkyl verknüpften unsubstituierten oder substituierten $(C_6-C_{14})$-Aryl-Rest, wie z.B. Benzyl, $\alpha$- und $\beta$-Naphthylmethyl, Halobenzyl und Alkoxybenzyl.

Unter Heteroaryl, Heterocyclus und unter einem Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom als Ringglieder werden Reste von Heteroaromaten verstanden, wie sie beispielsweise in Katritzky, Lagowski, Chemie der Heterocyclen, Berlin, Heidelberg 1968, definiert sind. Der Heteroaromaten-Rest kann durch einen, zwei oder drei, vorzugsweise einen oder zwei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl-alkyl.

Die Aminosäuren A und B und Formel I sind durch eine Amidbindung miteinander verknüpft. Es handelt sich um natürliche oder nichtnatürliche $\alpha$-Aminosäuren der L-, D-oder D,L-Konfiguration, vorzugsweise der L-Konfiguration.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder

Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in welchen

$R^1$ Wasserstoff, $(C_1-C_{10})$-Alkyl, das gegebenenfalls bis zu drei gleiche oder verschiedene Reste aus der Reihe Hydroxy, $(C_1-C_4)$-Alkoxy, $(C_1-C_4)$-Alkanoyloxy, Carboxy, $(C_1-C_4)$-Alkoxycarbonyl, Cl, Br, Amino, $(C_1-C_4)$-Alkylamino, Di-$(C_1-C_4)$-Alkylamino oder $(C_1-C_4)$-Alkoxycarbonylamino substituiert ist,
$(C_5-C_7)$-Cycloalkyl, $(C_6-C_{14})$-Aryl, das gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino und Trifluormethyl substituiert ist,
$(C_6-C_{14})$-Aryl-$(C_1-C_6)$-alkyl, worin der Arylteil gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino, $(C_1-C_4)$-Alkylamino, Di-$(C_1-C_4)$-alkylamino, Carboxy, Carboxymethoxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl, $(C_1-C_4)$-Alkoxycarbonylmethoxy, Carbamoyl, Sulfamoyl, $(C_1-C_4)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet und

A einen N-terminal über $-NR^6-$ mit -CO- und C-terminal über -CO- mit B verknüpften Rest einer natürlichen oder unnatürlichen Aminosäure aus der Reihe Phenylalanin, Histidin, Tyrosin, Tryptophan, β-2-Thienylalanin, β-3-Thienylalanin, β-2-Furylalanin, β-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 2-(2-Thienyl)-serin, (Z)-Dehydrophenylalanin oder (E)-Dehydrophenylalanin, und $R^6$ Wasserstoff bedeutet,

B einen N-terminal über $-NR^7-$ mit A und C-terminal über -CO- mit

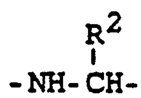

verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]-thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin oder (E)-Dehydrophenylalanin, (1,3-Dioxolan-2-yl)-alanin, N-Pyrrolylalanin, (1-, 3-oder 4-Pyrazolyl)-alanin, (4-Thiazolyl)alanin, (2-, 4-oder 5-Pyrimidyl)-alanin, Cyclopentylglycin, tert.Butylglycin oder Phenylserin und $R^7$ Wasserstoff bedeutet, und

$R^2$ Wasserstoff, $(C_1-C_5)$-Alkyl, $(C_5-C_6)$-Cycloalkyl-$(C_1-C_4)$-alkyl, $(C_6-C_{10})$-Aryl-$(C_1-C_4)$-alkyl oder (Heterocyclyl)-$(C_1-C_4)$-alkyl bedeutet, wobei der Heterocyclus 5 oder 6 Ringglieder, davon 1 oder 2 Schwefel- und/oder Sauerstoffatome, besitzt,

$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,

X wahlweise abwesend sein kann oder für -O-, -S-, $-CF_2-$, -CO- oder $-CHR^8-$ steht, worin

$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,

$R^4$ -OH, $-NH_2$ oder 5- oder 6-gliedriges Heteroaryl, das 1 oder 2 Stickstoffatome enthält, das teilweise oder vollständig hydriert sein kann, und das gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet,

p und q unabhängig voneinander 0, 1, 2, 3 oder 4 bedeuten,

$R^5$ die gleiche Bedeutung wie $R^1$ besitzt oder aber zusammen mit $R^1$ und dem sie verbindenden

Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino, ($C_1$-$C_6$)-Alkylamino-($C_1$-$C_6$)-alkyl, Di-($C_1$-$C_6$)-alkylamino oder Di-($C_1$-$C_6$)-alkylamino-($C_1$-$C_6$)-alkyl substituiert sein kann, bildet,
sowie deren physiologisch verträgliche Salze.

Besonders bevorzugt sind Verbindungen der Formel I, in welchen
$R^1$ Wasserstoff, ($C_1$-$C_{10}$)-Alkyl, das gegebenenfalls bis zu drei gleiche oder verschiedene Reste aus der Reihe Hydroxy, Carboxy, Amino, ($C_1$-$C_4$)-Alkylamino, Di-($C_1$-$C_4$)-alkylamino oder ($C_1$-$C_4$)-Alkoxycarbonylamino substituiert ist,
Phenyl, das gegebenenfalls durch einen oder zwei Reste aus der Reihe F, Cl, Hydroxy, Amino oder Trifluormethyl substituiert ist,
Phenyl-($C_1$-$C_4$)-alkyl, worin der Phenylteil gegebenenfalls durch einen oder zwei gleiche oder verschiedene Reste aus der Reihe F, Cl, Hydroxy, Amino, ($C_1$-$C_4$)-Alkylamino, Di-($C_1$-$C_4$)-alkylamino, Carboxy, Amino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl substituiert ist, bedeutet,
A einen N-terminal über -$NR^6$- mit -CO- und C-terminal über -CO- mit B verknüpften Rest einer Aminosäure aus der Reihe Phenylalanin, Tyrosin, $\beta$-2-Thienylalanin, $\beta$-3-Thienylalanin, 4-Chlorphenylalanin, O-Methyltyrosin, 1-Naphthylalanin, 2-Naphthylalanin, 2-Fluorphenylalanin, 3-Fluorphenylalanin, 4-Fluorphenylalanin und
$R^6$ Wasserstoff bedeutet,
B einen N-terminal über -$NR^7$- mit A und C-terminal über -CO- mit -NH-$CHR^2$- verknüpften Rest einer Aminosäure aus der Reihe Phenylalanin, Histidin, Leucin, $\beta$-2-Thienylalanin, $\beta$-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)-alanin, (1-, 3- oder 4-Pyrazolyl)-alanin, 4-Thiazolylalanin, (2-, 4- oder 5-Pyrimidyl)-alanin und
$R^7$ Wasserstoff bedeutet,
$R^2$ Isobutyl, Cyclohexylmethyl, Benzyl oder (1,3-Dithiolan-2-yl)-methyl bedeutet,
$R^3$ Wasserstoff bedeutet,
X wahlweise abwesend sein kann oder für -S-, -CO- oder -$CHR^8$- steht, worin
$R^8$ Wasserstoff, Hydroxy, ($C_1$-$C_5$)-Alkoxy oder Fluor bedeutet,
$R^4$ 2-Imidazolyl, 4-Imidazolyl, 5-Imidazolyl, 2-Pyridyl, 3-Pyridyl, 4-Pyridyl, 2-Piperidyl, 3-Piperidyl, 4-Piperidyl, 1-Piperazinyl oder 2-Piperazinyl bedeutet, wobei die genannten heterocyclischen Reste jeweils mit ein oder zwei gleichen oder verschiedenen Resten aus der Reihe Fluor, Methoxy, Methyl, Ethyl, ($C_1$-$C_4$)-Alkoxycarbonyl, Amino oder Trifluormethyl substituiert sein können,
p und q unabhängig voneinander 0, 1 oder 2 bedeuten,
$R^5$ die gleiche Bedeutung wie $R^1$ besitzt oder aber zusammen mit $R^1$ und dem sie verbindenden Stickstoffatom einen Rest aus der Reihe Pyrrolidinyl, Piperidyl, Morpholinyl, Thiomorpholinyl, Piperazinyl, Homopiperazinyl oder Hexahydroazepinyl bedeutet, wobei die genannten Reste jeweils mit ein oder zwei gleichen oder verschiedenen Substituenten aus der Reihe Methyl, Ethyl, Propyl, Isopropyl, n-Butyl, s-Butyl, Isobutyl, tert.Butyl, Hydroxy, Hydroxymethyl, Hydroxyethyl, Methoxy, Carboxy, Carboxymethyl, Carboxyethyl, Methoxycarbonyl, Ethoxycarbonyl, ($C_1$-$C_2$)-Alkoxycarbonyl-($C_1$-$C_4$)-alkyl, Fluor, Chlor, Brom, Amino, Amino-($C_1$-$C_4$)-alkyl, ($C_1$-$C_4$)-Alkylamino-, ($C_1$-$C_4$)-Alkylamino-($C_1$-$C_4$)-alkyl, Di-($C_1$-$C_4$)-alkylamino oder Di-($C_1$-$C_4$)-alkylamino-($C_1$-$C_4$)-alkyl substituiert sein können,
sowie deren physiologisch verträgliche Salze.

Die Verbindungen der Formel I können hergestellt werden, indem man eine Verbindung der Formel II

$$\text{H-A-B-NH-}\overset{R^2}{\overset{|}{\text{CH}}}\text{-}\overset{OH}{\overset{|}{\text{CH}}}\text{-}\overset{R^3}{\overset{|}{\text{CH}}}\text{-}(CH_2)_p\text{-X-}(CH_2)_q\text{-}R^4 \qquad (II)$$

in welcher $R^2$, $R^3$, $R^4$, A, B, X, p und q die gleiche Bedeutung wie in der Formel I besitzen, nacheinander zunächst mit einem Kohlensäurederivat der Formel III

$$R^9\text{-}\overset{O}{\overset{\|}{C}}\text{-}R^{10} \qquad (III),$$

worin $R^9$ und $R^{10}$ gleich oder verschieden unabhängig voneinander Halogen, ($C_1$-$C_7$)-Alkoxy, ($C_6$-$C_{12}$)-Aryloxy, ($C_1$-$C_7$)-Alkylthio, ($C_6$-$C_{12}$)-Arylthio oder einen Rest Het-oder Het-O- bedeutet, wobei Het ein

mono- oder bicyclischer Heterocyclus sein kann, oder worin $R^9$ und $R^{10}$ gemeinsam mit der C=O-Gruppe einem mono- oder bicyclischen Heterocyclus vom Typ

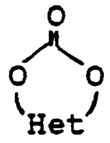 oder 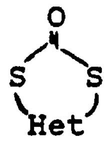 angehören,

und anschließend mit einem Amin der allgemeinen Formel IV

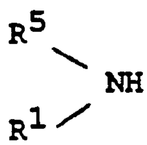 (IV)

worin $R^1$ und $R^5$ die gleiche Bedeutung wie in der Formel I besitzen, umsetzt, gegebenenfalls temporär eingeführte Schutzgruppen wieder abspaltet und die so erhaltene Verbindung gegebenenfalls in ihr physiologisch verträgliches Salz überführt.

Die Formel III steht vorzugsweise für Phosgen, 1,1′-Carbonyldiimidazol, 1,1′-Carbonyldi-(1,2,4)-triazol, Di-(N-succinimidyl)-carbonat, Di-(1-benzotriazolyl)-carbonat, N,N′-Carbonyl-bis-(2-methylimidazol) oder 4,6-Diphenylthieno[3,4-d]-1,3-dioxol-2-on-5,5-dioxid (Steglich-Reagenz).

Die Umsetzung wird bevorzugt in einem inerten organischen Lösungsmittel, wie z.B. Toluol, Methylenchlorid, Chloroform, Tetrahydrofuran, Dimethylformamid oder Acetonitril, ausgeführt.
Gegebenenfalls wird in Gegenwart einer Hilfsbase, wie z.B. Kalimcarbonat, Natriumcarbonat, Triethylamin, Pyridin, 1,5-Diazabicyclo-[5.4.0]-undec-5-en oder 1,5-Diazabicyclo-[4.3.0]-non-5-en, gearbeitet. Bevorzugt ist Pyridin,

Die Temperaturen können beispielsweise zwischen -80 °C und dem Siedepunkt des jeweiligen Lösungsmittels liegen, bevorzugt aber zwischen -80 °C und +50 °C.

Die Verbindungen der Formel I können auch erhalten werden, indem man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Die Verbindungen der Formel II werden erhalten aus Verbindungen der Formel V

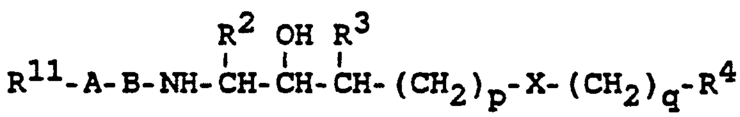 (V)

worin $R^2$, $R^3$, $R^4$, A, B, X, p und q die gleiche Bedeutung wie in Formel I besitzen und worin $R^{11}$ eine leicht abzuspaltende Amino-Schutzgruppe bedeutet, vorzugsweise tert.Butyloxycarbonyl oder Benzyloxycarbonyl, durch Abspaltung dieser Schutzgruppe unter den üblichen Bedingungen, z.B. durch saure oder alkalische Hydrolyse oder Hydrogenolyse, gegebenenfalls unter temporären Schutz der Hydroxyfunktion.

Die Verbindung der Formeln III und IV sind literaturbekannt und großenteils käuflich. Die Verbindungen der Formel V sind aus der Europäischen Patentanmeldung 255 082 bekannt bzw. auf analogem Wege aus den entsprechenden Ausgangsmaterialien erhältlich.

Fragmente einer Verbindung der Formel I mit einer endständigen Carboxylgruppe besitzen die nachstehenden Formeln VIa)-VIb):

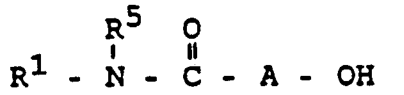 (VIa)

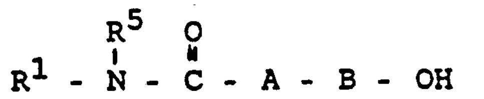 (VIb),

worin $R^1$, $R^5$, A und B die gleiche Bedeutung wie in der Formel I besitzen.

Fragmente einer Verbindung der Formel I mit einer endständigen Aminogruppe besitzen die nachstehenden Formeln VIIa)-VIIb):

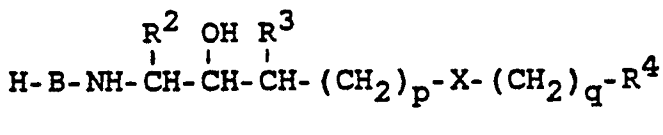 (VIIa)

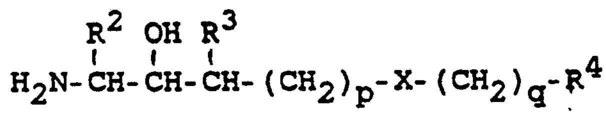 (VIIb)

worin $R^2$, $R^3$, $R^4$, B, X, p und q die gleiche Bedeutung wie in der Formel I besitzen.

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:

Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel VIIb erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher in einer Aldolanalogen Addition an einen entsprechenden Heteroarylalkyl-Baustein gekuppelt wird und nach Abspaltung der N-Schutzgruppe Aminoalkohole der Formel VIIb) ergibt. Alternativ dazu werden in an sich bekannter Weise aus den geschützten Aminoaldehyden über die Allylamine die Epoxide hergestellt. Diese können entweder direkt mit den entsprechenden Arylalkyl-Nucleophilen umgesetzt werden, oder man öffnet das Epoxid zunächst mit Trimethylsilylchlorid und NaI in Acetonitril, spaltet den Silylether mit CsF und schützt das Iodid mit 2,2-Dimethoxypropan unter Säurekatalyse als Oxazolidin. Dieses Iodid kann mit weniger reaktiven Nucleophilen umgesetzt werden. Zur Synthese der um eine $CH_2$-Gruppe verlängerten Arylalkyl-substituierten Aminoalkoholen geht man zum Beispiel von Boc-ACHPA-OEt (hergestellt nach J. Med. Chem. 28, 1779 (1985)) aus. Zunächst erfolgt N,O-Schützung, dann die Reduktion der Esterfunktion und schließlich die Umwandlung der Hydroxyfunktion in ein Bromid, das unter analogen Bedingungen wie die bereits genannten Elektrophile mit Arylalkyl-Nucleophilen umgesetzt werden kann. In Frage kommende Arylalkyl-Nucleophile sind z.B. Acetylimine und Acetylhydrazone. Weitere Verbindungen der Arylalkyl-substituierten Aminoalkohole mit verlängerter(n) $CH_2$-Gruppe(n) können nach den allgemein üblichen Methoden zur Kettenverlängerung erhalten werden. Falls der gewählte Syntheseweg zu Diastereomeren bezüglich des OH tragenden Zentrums führt, können diese in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et al., J. org. Chem. 34, 2543 (1969)).

Die Herstellung N-geschützter Aminosäurealdehyde erfolgt nach B. Castro et al. (Synthesis 1983, 676).

Die Addition der Arylalkyl-Nucleophile an die genannten N-geschützten Elektrophile (bevorzugterweise N-tert.-Butoxycarbonyl- und Benzyloxycarbonyl-Schutzgruppen) erfolgt in einem gegenüber Basen inerten Lösungsmittel wie Ether, THF, Toluol, DMF, DMSO oder Dimethoxyethan.

Als Basen zur Deprotonierung der Heteroarylalkyl-Komponente können Alkalimetallalkoholate, wie

Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die zur Herstellung von Verbindungen der Formel I erforderlichen Vor- und Nachoperationen wie Einführung und Abspaltung von Schutzgruppen sind literaturbekannt und z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäuren A und B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf, insbesondere hemmen sie Aspartylproteasen wie das Renin und virale Proteasen wie die HIV-Protease.

Renin ist ein proteolytisches Enzym aus der Klasse der Aspartylproteasen, welches als Folge verschiedener Stimuli (Volumendepletion, Natriummangel, $\beta$-Rezeptorenstimulation) von den juxtaglomerulären Zellen der Niere in den Blutkreislauf sezerniert. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkonstriktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37°C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4°C, 15 min; wiederholen, falls erforderlich) wird das Plasma vorsichtig abpipettiert und in geeigneten Portionen bei -30°C eingefroren. Für den Test werden nur Plasmen mit ausreichend hoher Reninaktivität verwendet. Plasmen mit niedriger Reninaktivität werden durch eine Kältebehandlung (-4°C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:
Inkubationsansatz: 1000 µl Plasma (bei 0-4°C aufgetaut)
100 µl Phosphatpuffer (pH 7,4) Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO

entsprechend verdünnt; der Inkubationsansatz enthält max. 1 % DMSO.

Die Ansätze werden in Eis gemischt und für 1 Stunde zur Inkubation in ein Wasserbad (37 °C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25 °C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten, wie zum Beispiel Rhesus-Affen, herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1 - 50 mg/kg. Die in der vorliegenden Erfindung beschriebenen Verbindungen der allgemeinen Formel I können als Antihypertensiva sowie zur Behandlung der Herzinsuffizienz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirking, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-8}$ Mol/l.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel I zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen oder auch eine Mischung aus den verschiedenen genannten Lösungsmitteln.

Verzeichnis der verwendeten Abkürzungen:

Boc tert.-Butoxycarbonyl
DC Dünnschichtchromatographie
DCC Dicyclohexylcarbodiimid
DCI Desorption Chemical Ionisation
DNP 2,4-Dinitrophenyl
EI Electron Impact
FAB Fast atom bombardment
HOBt 1-Hydroxybenzotriazol
M Molekularpeak
MeOH Methanol
MS Massenspektrum
R.T. Raumtemperatur
Schmp. Schmelzpunkt
Thi β-2-Thienylalanin

Die sonstigen für Aminosäuren verwendeten Abkürzungen entsprechen dem in der Peptidchemie üblichen Drei-Buchstaben-Code, wie er z.B. in Eur. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen der Erläuterung der vorliegenden Erfindung, ohne diese darauf zu beschränken.

**Beispiel 1**

1-Cyclohexyl-6-(2-pyridyl)-2S-[N-[N-(piperazin-1-yl-carbonyl)-L-phenylalanyl-L-norvalyl]amino]-3S-hexanol

1a) Boc-Phe-Nva-OMe

15,0 g (0,056 mol) Boc-Phe-OH und 9,4 g (0,056 mol) Nva-OMe-hydrochlorid werden in 250 ml absolutem Methylenchlorid gelöst. Dazu tropft man unter Eiskühlung zunächst 38,6 ml (0,28 mol) absolutes Triethylamin und dann 36,4 ml Propanphosphonsäureanhydrid (50 % in Methylenchlorid). Die Reaktionslösung wird 3 Stunden bei Raumtemperatur nachgerührt und über Nacht stehengelassen. Zur Hydrolyse gibt man auf Eiswasser, rührt 2 Stunden kräftig nach, trennt die organische Phase ab und wäscht sie mit 10 %iger Citronensäurelösung, gesättigter Natriumhydrogencarbonatlösung und Wasser, trocknet über Natriumsulfat und engt ein.
Ausbeute: 20,8 g (98 %) der Titelverbindung
$R_f$ (Methylenchlorid/MeOH 9:1) = 0,69
MS (DCI) = 378 (M+1)

1b) Boc-Phe-Nva-OH

20,1 g (0,053 mol) Boc-Phe-Nva-OMe (Beispiel 1a) werden in 30 ml Wasser und 30 ml Dioxan suspendiert. Bei Raumtemperatur werden 2,5 g (0,106 mol) Lithiumhydroxid eingetragen und 3 Stunden bei Raumtemperatur nachgerührt. Die Reaktionslösung wird mit 10 %iger Natriumhydrogensulfatlösung angesäuert und das Produkt abgesaugt und mit Diisopropylether verrührt.
Ausbeute: 19,1 g (98 %)
MS (DCI) = 364 (M+1)

1c) 2S-Amino-1-cyclohexyl-6-(2-pyridyl)-3S-hexanol

Zu 214 mg (0,513 mmol) 3-Boc-4S-cyclohexylmethyl-2,2-dimethyl-5-(3-(2-pyridyl)-propyl)-oxazoldin in 10 ml Dimethoxymethan tropft man im Eisbad 5 ml HCl-gesättigtes Dimethoxyethan und rührt eine Stunde bei 0 °C und 5 Stunden bei Raumtemperatur nach. Die Reaktionslösung wird im Vakuum eingeengt und noch zweimal mit Toluol abgeraucht.
Ausbeute: 179 mg der Titelverbindung als Dihydrochlorid

1d) 1-Cyclohexyl-6-(2-pyridyl)-2S-[N-(Boc-Phe-Nva)-amino]-3S-hexanol

1,7 g (3,36 mmol) 2S-Amino-1-cyclohexyl-6-(2-pyridyl)-3S-hexanol-dihydrochlorid (hergestellt analog Beispiel 1c) werden in 10 ml absolutem Dimethylformamid gelöst und bei Raumtemperatur nacheinander 1,22 g (3,36 mmol) Boc-Phe-Nva-OH (Beispiel 1b) und 0,9 g HOBt zugegeben. Dann wird auf 4 °C abgekühlt und bei dieser Temperatur 2 ml (15,6 mmol) N-Ethylmorpholin sowie 0,69 g (3,36 mmol) DCC zugesetzt. Das Reaktionsgemisch wird 1 Stunde im Eisbad und 5 Stunden bei R.T. gerührt und 3 Tage stehengelassen. Man saugt vom Niederschlag ab, gibt das Filtrat auf Eiswasser und extrahiert mehrmals mit Essigester. Die vereinigten organischen Phasen werden mit gesättigter Natriumbicarbonatlösung, Wasser und gesättigter Natriumchloridlösung gewaschen, getrocknet, eingeengt und das Rohprodukt durch Säulenchromatographie an Kieselgel (Laufmittel Methylenchlorid/Methanol 100:0, 98:2, 95:5) gereinigt. Man erhält 0,8 g der Titelverbindung.
$R_f$ (Methylenchlorid/MeOH 9:1) = 0,53
MS (FAB) = 623 (M+1)

1e) 1-Cyclohexyl-6-(2-pyridyl)-2S-[N-(H-Phe-Nva)-amino]-3S-hexanol

Aus 97 mg 1-Cyclohexyl-6-(2-pyridyl)-2S-[N-(Boc-Phe-Nva)-amino]-3S-hexanol (Beispiel 1d) erhält man analog dem in Beispiel 1c) angegebenen Verfahren 92 mg der Titelverbindung als Dihydrochlorid.

1f) 1-Cyclohexyl-6-(2-pyridyl)-2S-[N-[N-(piperazin-1-yl-carbonyl)-Phe-Nva]amino]-3S-hexanol

Eine Lösung von 118 mg (0,16 mmol) 1-Cyclohexyl-6-(2-pyridyl)-2S-[N-(H-Phe-Nva)-amino]-3S-hexanol (Beispiel 1e) in 10 ml absolutem Methylenchlorid wird bei -75 °C zu einer Lösung von 125 mg (0,32 mmol) Di(benzotriazoyl)carbonat und 26 µl (0,32 mmol) Pyridin in 10 ml absolutem Methylenchlorid getropft, 2 Stunden bei dieser Temperatur und 1 Stunde bei R.T. nachgerührt. Dann wird die Lösung erneut auf -75 °C abgekühlt und 69 mg (0,8 mmol) Piperazin zugegeben. Nach einer Stunde bei -75 °C läßt man langsam aufwärmen und läßt über Nacht bei R.T. stehen. Die Reaktionslösung wird eingeengt, der Rückstand in Essigester aufgenommen, mit gesättigter Natriumhydrogencarbonatlösung, Wasser und gesättigter Natriumchloridlösung gewaschen, getrocknet, eingeengt und das Rohprodukt (106 mg) durch Mitteldruck-Säulenchromatographie an Kieselgel (Laufmittelgradient Methylenchlorid/MeOH 99:1 bis 1:1) gereinigt. Man erhält 25,7 mg der Titelverbindung.
$R_f$ (Methylenchlorid/MeOH 9:1) = 0,03
MS (FAB) = 635 (M+1)

**Beispiel 2**

1-Cyclohexyl-6-(2-pyridyl)-2S-[N-[N-(morpholinocarbonyl)-L-phenylalanyl-L-norvalyl]amino]-3S-hexanol

Aus 108 mg (0,17 mmol) 1-Cyclohexyl-6-(2-pyridyl)-2S-[N-(H-Phe-Nva)-amino]-3S-hexanol (Beispiel 1e), 133 mg (0,34 mmol) Di-(benzotriazolyl)carbonat und 74 µl (0,85 mmol) Morpholin erhält man analog dem in Beispiel 1f) beschriebenen Verfahren 56,6 mg der Titelverbindung.
$R_f$ (Methylenchlorid/MeOH 9:1) = 0,39
MS (FAB) = 636 (M+1)

Unter Verwendung geeigneter Ausgangsmaterialien und unter Anwendung der in den Beispielen 1 bis 2

beschriebenen Verfahren werden die folgenden Verbindungen erhalten.

**Beispiel 3**

1-Cyclohexyl-6-(2-pyridyl )-2S-[N-[N-(dimethylaminocarbonyl)-L-phenylalanyl-L-norvalyl{$^{80}$♀]amino]-3S-hexanol;

MS (FAB) = 594 (M+1)

**Beispiel 4**

1-Cyclohexyl-6-(2-pyridyl)-2S-[N-[N-(diethylaminocarbonyl)-L-phenylalanyl-L-norvalyl]amino]-3S-hexanol;

MS (FAB) = 622 (M+1)

**Beispiel 5**

1-Cyclohexyl-6-(2-pyridyl)-2S-[N-[N-(4-methylpiperazin-1-yl-carbonyl)-L-phenylalanyl-L-norvalyl]amino]-3S-hexanol;

MS (FAB) = 649 (M+1)

**Beispiel 6**

1-Cyclohexyl-6-(2-pyridyl)-2S-[N-[N-(thiomorpholinocarbonyl)-L-phenylalanyl-L-norvalyl]amino]-3S-hexanol;

MS (FAB) = 652 (M+1)

**Beispiel 7**

1-Cyclohexyl-6-(2-pyridyl)-2S-[N-[N-(1-piperidylcarbonyl)-L-phenylalanyl-L-norvalyl]amino]-3S-hexanol;

MS (FAB) = 634 (M+1)

**Beispiel 8**

1-Cyclohexyl-6-(2-pyridyl)-2S-[N-[N-(4-tert.butyloxy-carbonylpiperazin-1-yl-carbonyl)-L-phenylalanyl-L-norvalyl]-amino]-3S-hexanol;

MS (FAB) = 735 (M+1)

**Beispiel 9**

1-Cyclohexyl-6-(2-pyridyl)-2S-[N-[N-(dimethylaminocarbonyl)-L-phenylalanyl-L-histidyl]amino]-3S-hexanol;

MS (FAB) = 632 (M+1)

**Beispiel 10**

1-Cyclohexyl-6-(2-pyridyl)-2S-[N-[N-(diethylaminocarbonyl)-L-phenylalanyl-L-histidyl]amino]-3S-hexanol;

MS (FAB) = 660 (M+1)

**Beispiel 11**

1-Cyclohexyl-6-(2-pyridyl)-2S-[N-[N-(morpholinocarbonyl)-L-phenylalanyl-L-histidyl] amino]-3S-hexanol;

MS (FAB) = 674 (M+1)

**Beispiel 12**

1-Cyclohexyl-6-(2-pyridyl)-2S-[N-[N-(piperazin-1-yl-carbonyl)-L-phenylalanyl-L-histidyl]amino]-3S-hexanol;

MS (FAB) = 673 (M+1)

**Beispiel 13**

1-Cyclohexyl-6-(2-pyridyl)-2S-[N-[N-(4-methylpiperazin-1-yl-carbonyl)-L-phenylalanyl-L-histidyl]amino]-3S-hexanol;

MS (FAB) = 687 (M+1)

**Beispiel 14**

1-Cyclohexyl-6-(2-pyridyl)-2S-[N-[N-(thiomorpholinocarbonyl)-L-phenylalanyl-L-histidyl]amino]-3S-hexanol;

MS (FAB) = 690 (M+1)

**Beispiel 15**

1-Cyclohexyl-6-(2-pyridyl)-2S-[N-[N-(1-piperidylcarbonyl)-L-phenylalanyl-L-histidyl]amino]-3S-hexanol;

MS (FAB) = 672 (M+1)

**Beispiel 16**

1-Cyclohexyl-6-(2-pyridyl)-2S-[N-[N-(4-tert.butyloxycarbonylpiperazin-1-yl-carbonyl)-L-phenylalanyl-L-histidyl]amino]-3S-hexanol;

MS (FAB) = 773 (M+1)

**Beispiel 17**

1-Cyclohexyl-6-(2-pyridyl)-2S-[N-[N-(morpholinocarbonyl)--L-(β-2-thienylalanyl)-L-norvalyl]amino]-3S-hexanol;

MS (FAB) = 642 (M+1)

**Beispiel 18**

1-Cyclohexyl-6-(2-pyridyl)-2S-[N-[N-(piperazin-1-yl-carbonyl)-L-(β-2-thienylalanyl)-L-norvalyl]amino]-3S-hexanol;

MS (FAB) = 641 (M+1)

**Beispiel 19**

1-Cyclohexyl-6-(2-pyridyl)-2S-[N-[N-(morpholinocarbonyl)-L-(β-2-thienylalanyl)-L-histidyl]amino]-3S-hexanol;

MS (FAB) = 680 (M+1)

**Beispiel 20**

1-Cyclohexyl-6-(2-pyridyl)-2S-[N-[N-(piperazin-1-yl-carbonyl)-L-(β-2-thienylalanyl)-L-histidyl]amino]-3S-hexanol;

MS (FAB) = 679 (M+1)

**Beispiel 21**

1-Phenyl-6-(2-pyridyl)-2S-[N-[N-(morpholinocarbonyl)-L-phenylalanyl-L-norvalyl]amino]-3S-hexanol;

MS (FAB) = 635 (M+1)

**Beispiel 22**

2-Methyl-8-(2-pyridyl)-4S-[N-[N-(morpholinocarbonyl)-L-phenylalanyl-L-histidyl]amino]-5S-octanol;

MS (FAB) = 634 (M+1)

**Beispiel 23**

1-Cyclohexyl-5-fluor-6-(2-pyridyl)-2S-[N-[N-(morpholinocarbonyl)-L-phenylalanyl-L-norvalyl]amino]-3S-hexanol;

MS (FAB) = 654 (M+1)

**Beispiel 24**

1-Cyclohexyl-5-fluor-6-(2-pyridyl)-2S-[N-[N-(morpholinocarbonyl)-L-phenylalanyl-L-histidyl]amino]-3S-hexanol;

MS (FAB) = 692 (M+1)

**Beispiel 25**

1-Cyclohexyl-6-(1-methylimidazol-2-yl)-2S-[N-[N-(morpholinocarbonyl)-L-phenylalanyl-L-norvalyl]amino]-3S-hexanol;

MS (FAB) = 641 (M+1)

**Beispiel 26**

1-Cyclohexyl-6-(1-methylimidazol-2-yl)-2S-[N-[N-(morpholinocarbonyl)-L-phenylalanyl-L-histidyl]amino]-3S-hexanol.

MS (FAB) = 679 (M+1)

## Ansprüche

1. Verbindungen der Formel I

$$R^1-\overset{R^5}{\underset{|}{N}}-\overset{O}{\overset{\|}{C}}-A-B-NH-\overset{R^2}{\overset{|}{C}H}-\overset{OH}{\overset{|}{C}H}-\overset{R^3}{\overset{|}{C}H}-(CH_2)_p-X-(CH_2)_q-R^4 \qquad (I)$$

in welcher
$R^1$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,
($C_3$-$C_8$)-Cycloalkyl, ($C_3$-$C_8$)-Cycloalkyl-($C_1$-$C_6$)-alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,
($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet,
A einen N-terminal über -$NR^6$ mit -CO- und C-terminal über -CO- mit B verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert.Butylglycin, Phenylserin und $R^6$ Wasserstoff, ($C_1$-$C_6$)-Alkyl, Formyl, ($C_1$-$C_6$)-Alkoxycarbonyl oder Benzyloxycarbonyl bedeutet,
B einen N-terminal über -$NR^7$- mit A und C-terminal über -CO- mit

$$-NH-\overset{R^2}{\overset{|}{C}H}-$$

verknüpften Rest einer natürlichen oder unnatürlichen Aminosäure, wie für A definiert, und $R^7$ Wasserstoff, $(C_1-C_6)$-Alkyl, Formyl, $(C_1-C_6)$-Alkoxycarbonyl oder Benzyloxycarbonyl bedeutet,
$R^2$ Wasserstoff, $(C_1-C_{10})$-Alkyl, $(C_4-C_7)$-Cycloalkyl, $(C_4-C_7)$-Cycloalkyl-$(C_1-C_6)$-alkyl, $(C_6-C_{14})$-Aryl, $(C_6-C_{14})$-Aryl-$(C_1-C_6)$-alkyl oder (Heterocyclyl)-$(C_1-C_6)$alkyl bedeutet, wobei der Heterocyclus 4-7 Ringglieder, davon 1 oder 2 Schwefel-und/oder Sauerstoffatome, besitzt,
$R^3$ Wasserstoff, $(C_1-C_{10})$-Alkyl, $(C_6-C_{14})$-Aryl oder $(C_6-C_{14})$-Aryl-$(C_1-C_6)$-alkyl bedeutet,
X wahlweise abwesend sein kann oder für -O-, -S-, $-CF_2-$, -CO- oder $-CHR^8-$ steht, worin
$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,
$R^4$ -OH, $-NH_2$ oder Heteroaryl, das auch teilweise oder vollständig hydriert sein kann, und das gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet,
p und q unabhängig voneinander 0, 1, 2, 3 oder 4 bedeuten,
$R^5$ die gleiche Bedeutung wie $R^1$ besitzt oder aber zusammen mit $R^1$ und dem sie verbindenden Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet,
sowie deren physiologisch verträgliche Salze.

2. Verbindungen der Formel I, gemäß Anspruch 1, dadurch gekennzeichnet, daß
$R^1$ Wasserstoff, $(C_1-C_{10})$-Alkyl, das gegebenenfalls bis zu drei gleiche oder verschiedene Reste aus der Reihe Hydroxy, $(C_1-C_4)$-Alkoxy, $(C_1-C_4)$-Alkanoyloxy, Carboxy, $(C_1-C_4)$-Alkoxycarbonyl, Cl, Br, Amino, $(C_1-C_4)$-Alkylamino, Di-$(C_1-C_4)$-Alkylamino oder $(C_1-C_4)$-Alkoxycarbonylamino substituiert ist,
$(C_5-C_7)$-Cycloalkyl, $(C_6-C_{14})$-Aryl, das gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino und Trifluormethyl substituiert ist,
$(C_6-C_{14})$-Aryl-$(C_1-C_6)$-alkyl, worin der Arylteil gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino, $(C_1-C_4)$-Alkylamino, Di$(C_1-C_4)$-alkylamino, Carboxy, Carboxymethoxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl, $(C_1-C_4)$-Alkoxycarbonylmethoxy, Carbamoyl, Sulfamoyl, $(C_1-C_4)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet und
A einen N-terminal über $-NR^6-$ mit -CO- und C-terminal über -CO- mit B verknüpften Rest einer natürlichen oder unnatürlichen Aminosäure aus der Reihe Phenylalanin, Histidin, Tyrosin, Tryptophan, β-2-Thienylalanin, β-3-Thienylalanin, β-2-Furylalanin, β-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 2-(2-Thienyl)-serin, (Z)-Dehydrophenylalanin oder (E)-Dehydrophenylalanin, und $R^6$ Wasserstoff bedeutet,
B einen N-terminal über $-NR^7-$ mit A und C-terminal über -CO- mit

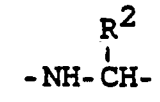

verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]-thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin oder (E)-Dehydrophenylalanin, (1,3-Dioxolan-2-yl)-alanin, N-Pyrrolylalanin, (1-, 3-oder 4-Pyrazolyl)-alanin, (4-Thiazolyl)alanin, (2-, 4-oder 5-Pyrimidyl)-alanin, Cyclopentylglycin, tert.Butylglycin oder Phenylserin und $R^7$ Wasserstoff bedeutet, und

$R^2$ Wasserstoff, $(C_1-C_5)$-Alkyl, $(C_5-C_6)$-Cycloalkyl-$(C_1-C_4)$-alkyl, $(C_6-C_{10})$-Aryl-$(C_1-C_4)$-alkyl oder (Heterocyclyl)-$(C_1-C_4)$-alkyl bedeutet, wobei der Heterocyclus 5 oder 6 Ringglieder, davon 1 oder 2 Schwefel- und/oder Sauerstoffatome, besitzt,

$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,

X wahlweise abwesend sein kann oder für -O-, -S-, $-CF_2-$, -CO- oder $-CHR^8-$ steht, worin

$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,

$R^4$ -OH, $-NH_2$ oder 5- oder 6-gliedriges Heteroaryl, das 1 oder 2 Stickstoffatome enthält, das teilweise oder vollständig hydriert sein kann, und das gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet,

p und q unabhängig voneinander 0, 1, 2, 3 oder 4 bedeuten,

$R^5$ die gleiche Bedeutung wie $R^1$ besitzt oder aber zusammen mit $R^1$ und dem sie verbindenden Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet,

sowie deren physiologisch verträgliche Salze.

3. Verbindungen der Formel I gemäß Anspruch 1 und/oder 2, dadurch gekennzeichnet, daß

$R^1$ Wasserstoff, $(C_1-C_{10})$-Alkyl, das gegebenenfalls bis zu drei gleiche oder verschiedene Reste aus der Reihe Hydroxy, Carboxy, Amino, $(C_1-C_4)$-Alkylamino, Di-$(C_1-C_4)$-alkylamino oder $(C_1-C_4)$-Alkoxycarbonylamino substituiert ist,

Phenyl, das gegebenenfalls durch einen oder zwei Reste aus der Reihe F, Cl, Hydroxy, Amino oder Trifluormethyl substituiert ist,

Phenyl-$(C_1-C_4)$-alkyl, worin der Phenylteil gegebenenfalls durch einen oder zwei gleiche oder verschiedene Reste aus der Reihe F, Cl, Hydroxy, Amino, $(C_1-C_4)$-Alkylamino, Di-$(C_1-C_4)$-alkylamino, Carboxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl substituiert ist, bedeutet,

A einen N-terminal über $-NR^6-$ mit -CO- und C-terminal über -CO- mit B verknüpften Rest einer Aminosäure aus der Reihe Phenylalanin, Tyrosin, β-2-Thienylalanin, β-3-Thienylalanin, 4-Chlorphenylalanin, O-Methyltyrosin, 1-Naphthylalanin, 2-Naphthylalanin, 2-Fluorphenylalanin, 3-Fluorphenylalanin, 4-Fluorphenylalanin und $R^6$ Wasserstoff bedeutet,

B einen N-terminal über $-NR^7-$ mit A und C-terminal über -CO- mit $-NH-CHR^2-$ verknüppften Rest einer Aminosäure aus der Reihe Phenylalanin, Histidin, Leucin, β-2-Thienylalanin, β-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)-alanin, (1-, 3- oder 4-Pyrazolyl)-alanin, 4-Thiazolylalanin, (2-, 4- oder 5-Pyrimidyl)-alanin und $R^7$ Wasserstoff bedeutet,

$R^2$ Isobutyl, Cyclohexylmethyl, Benzyl oder (1,3-Dithiolan-2-yl)-methyl bedeutet,

$R^3$ Wasserstoff bedeutet,

X wahlweise abwesend sein kann oder für -S-, -CO- oder $-CHR^8-$ steht, worin

$R^8$ Wasserstoff, Hydroxy, $(C_1-C_5)$-Alkoxy oder Fluor bedeutet,

$R^4$ 2-Imidazolyl, 4-Imidazolyl, 5-Imidazolyl, 2-Pyridyl, 3-Pyridyl, 4-Pyridyl, 2-Piperidyl, 3-Piperidyl, 4-Piperidyl, 1-Piperazinyl oder 2-Piperazinyl bedeutet, wobei die genannten heterocyclischen Reste jeweils mit ein oder zwei gleichen oder verschiedenen Resten aus der Reihe Fluor, Methoxy, Methyl, Ethyl, $(C_1-C_4)$-Alkoxycarbonyl, Amino oder Trifluormethyl substituiert sein können,

p und q unabhängig voneinander 0, 1 oder 2 bedeuten,

$R^5$ die gleiche Bedeutung wie $R^1$ besitzt oder aber zusammen mit $R^1$ und dem sie verbindenden Stickstoffatom einen Rest aus der Reihe Pyrrolidinyl, Piperidyl, Morpholinyl, Thiomorpholinyl, Piperazinyl, Homopiperazinyl oder Hexahydroazepinyl bedeutet, wobei die genannten Reste jeweils mit ein oder zwei

gleichen oder verschiedenen Substituenten aus der Reihe Methyl, Ethyl, Propyl, Isopropyl, n-Butyl, s-Butyl, Isobutyl, tert.Butyl, Hydroxy, Hydroxymethyl, Hydroxyethyl, Methoxy, Carboxy, Carboxymethyl, Carboxyethyl, Methoxycarbonyl, Ethoxycarbonyl, $(C_1-C_2)$-Alkoxycarbonyl-$(C_1-C_4)$-alkyl, Fluor, Chlor, Brom, Amino, Amino-$(C_1-C_4)$-alkyl, $(C_1-C_4)$-Alkylamino-, $(C_1-C_4)$-Alkylamino-$(C_1-C_4)$-alkyl, Di-$(C_1-C_4)$-alkylamino oder Di-$(C_1-C_4)$-alkylamino-$(C_1-C_4)$-alkyl substituiert sein können,
sowie deren physiologisch verträgliche Salze.

4. Verfahren zur Herstellung von Verbindungen der Formel I der Ansprüche 1 bis 3, dadurch gekennzeichnet, daß man

a) eine Verbindung der Formel II

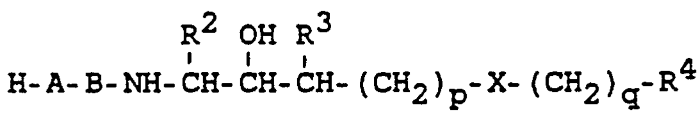 (II)

in welcher $R^2$, $R^3$, $R^4$, A, B, X, p und q die gleiche Bedeutung wie in der Formel I besitzen, nacheinander zunächst mit einem Kohlensäurederivat der Formel III

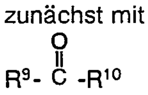 (III),

worin $R^9$ und $R^{10}$ gleich oder verschieden unabhängig voneinander Halogen, $(C_1-C_7)$-Alkoxy, $(C_6-C_{12})$-Aryloxy, $(C_1-C_7)$-Alkylthio, $(C_6-C_{12})$-Arylthio oder einen Rest Het-oder Het-O- bedeutet, wobei Het ein mono- oder bicyclischer Heterocyclus sein kann, oder worin $R^9$ und $R^{10}$ gemeinsam mit der C=O-Gruppe einem mono- oder bicyclischen Heterocyclus vom Typ

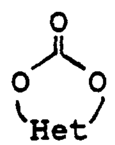 oder 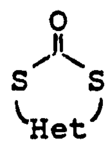 angehören,

und anschließend mit einem Amin der allgemeinen Formel IV

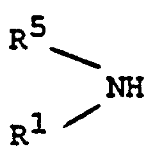 (IV)

worin $R^1$ und $R^5$ die gleiche Bedeutung wie in der Formel I besitzen, umsetzt, gegebenenfalls temporär eingeführte Schutzgruppen wieder abspaltet und die so erhaltene Verbindung gegebenenfalls in ihr physiologisch verträgliches Salz überführt.

b) ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

5. Verwendung einer Verbindung gemäß einem der Ansprüche 1 bis 3 als Heilmittel.

6. Verwendung einer Verbindung gemäß einem der Ansprüche 1 bis 3 als Heilmittel bei der Behandlung des Bluthochdrucks.

7. Verwendung einer Verbindung gemäß einem der Ansprüche 1 bis 3 als Heilmittel bei der Behandlung von Viruserkrankungen.

8. Pharmazeutisches Mittel enthaltend eine Verbindung gemäß einem der Ansprüche 1 bis 3.

Patentansprüche für folgende Vertragsstaaten: ES, GR

1. Verfahren zur Herstellung einer Verbindung der Formel I

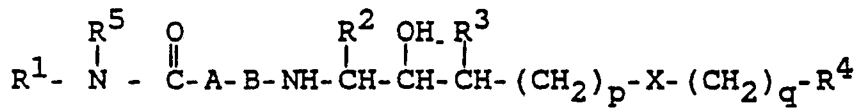

(I)

in welcher

$R^1$ Wasserstoff, $(C_1-C_{10})$-Alkyl, das gegebenenfalls ein-oder zweifach ungesättigt ist und das gegebenenfalls durch bis zu 3 gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, Cl, Br, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethyloxycarbonylamino substituiert ist,

$(C_3-C_8)$-Cycloalkyl, $(C_3-C_8)$-Cycloalkyl-$(C_1-C_6)$-alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist,

$(C_6-C_{14})$-Aryl-$(C_1-C_6)$-alkyl, worin der Arylteil gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet,

A einen N-terminal über $-NR^6$ mit -CO- und C-terminal über -CO- mit B verknüupften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo[b]thienylalanin, $\beta$-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert.Butylglycin, Phenylserin und $R^6$ Wasserstoff, $(C_1-C_6)$-Alkyl, Formyl, $(C_1-C_6)$-Alkoxycarbonyl oder Benzyloxycarbonyl bedeutet,

B einen N-terminal über $-NR^7$- mit A und C-terminal über -CO- mit

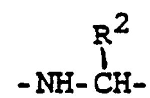

verknüpften Rest einer natürlichen oder unnatürlichen Aminosäure, wie für A definiert, und $R^7$ Wasserstoff, $(C_1-C_6)$-Alkyl, Formyl, $(C_1-C_6)$-Alkoxycarbonyl oder Benzyloxycarbonyl bedeutet,

$R^2$ Wasserstoff, $(C_1-C_{10})$-Alkyl, $(C_4-C_7)$-Cycloalkyl, $(C_4-C_7)$-Cycloalkyl-$(C_1-C_6)$-alkyl, $(C_6-C_{14})$-Aryl, $(C_6-C_{14})$-Aryl-$(C_1-C_6)$-alkyl oder (Heterocyclyl)-$(C_1-C_6)$ alkyl bedeutet, wobei der Heterocyclus 4-7 Ringglieder, davon 1 oder 2 Schwefel-und/oder Sauerstoffatome, besitzt,

$R^3$ Wasserstoff, $(C_1-C_{10})$-Alkyl, $(C_6-C_{14})$-Aryl oder $(C_6-C_{14})$-Aryl-$(C_1-C_6)$-alkyl bedeutet,

X wahlweise abwesend sein kann oder für -O-, -S-, $-CF_2$-, -CO- oder $-CHR^8$- steht, worin

$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,

$R^4$ -OH, $-NH_2$ oder Heteroaryl, das auch teilweise oder vollständig hydriert sein kann, und das gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet,

p und q unabhängig voneinander 0, 1, 2, 3 oder 4 bedeuten,

$R^5$ die gleiche Bedeutung wie $R^1$ besitzt oder aber zusammen mit $R^1$ und dem sie verbindenden Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino, ($C_1$-$C_6$)-Alkylamino-($C_1$-$C_6$)-alkyl, Di-($C_1$-$C_6$)-alkylamino oder Di-($C_1$-$C_6$)alkylamino-($C_1$-$C_6$)-alkyl substituiert sein kann, bildet,

sowie deren physiologisch verträgliche Salze, dadurch gekennzeichnet, daß

a) eine Verbindung der Formel II

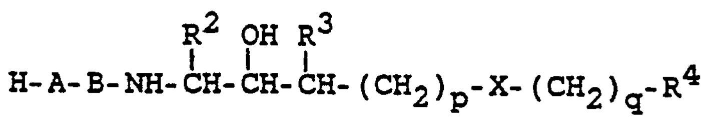

(II)

in welcher $R^2$, $R^3$, $R^4$, A, B, X, p und q die gleiche Bedeutung wie in der Formel I besitzen, nacheinander zunächst mit einem Kohlensäurederivat der Formel III

$R^9-\overset{O}{\overset{\|}{C}}-R^{10}$ (III),

worin $R^9$ und $R^{10}$ gleich oder verschieden unabhängig voneinander Halogen, ($C_1$-$C_7$)-Alkoxy, ($C_6$-$C_{12}$)-Aryloxy, ($C_1$-$C_7$)-Alkylthio, ($C_6$-$C_{12}$)-Arylthio oder einen Rest Het-oder Het-O- bedeutet, wobei Het ein mono- oder bicyclischer Heterocyclus sein kann, oder worin $R^9$ und $R^{10}$ gemeinsam mit der C=O-Gruppe einem mono- oder bicyclischen Heterocyclus vom Typ

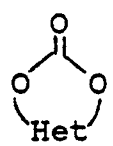 oder 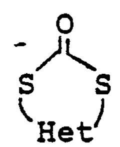 angehören,

und anschließend mit einem Amin der allgemeinen Formel IV

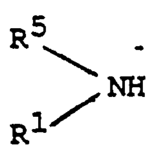

(IV)

worin $R^1$ und $R^5$ die gleiche Bedeutung wie in der Formel I besitzen, umsetzt, gegebenenfalls temporär eingeführte Schutzgruppen wieder abspaltet und die so erhaltene Verbindung gegebenenfalls in ihr physiologisch verträgliches Salz überführt, oder

b) ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß

$R^1$ Wasserstoff, ($C_1$-$C_{10}$)-Alkyl, das gegebenenfalls bis zu drei gleiche oder verschiedene Reste aus der Reihe Hydroxy, ($C_1$-$C_4$)-Alkoxy, ($C_1$-$C_4$)-Alkanoyloxy, Carboxy, ($C_1$-$C_4$)-Alkoxycarbonyl, Cl, Br, Amino, ($C_1$-$C_4$)-Alkylamino, Di-($C_1$-$C_4$)-Alkylamino oder ($C_1$-$C_4$)-Alkoxycarbonylamino substituiert ist,

($C_5$-$C_7$)-Cycloalkyl, ($C_6$-$C_{14}$)-Aryl, das gegebenenfalls durch einen oder zwei gleiche oder verschiedene Reste aus der Reihe F, Cl, Br, Hydroxy, ($C_1$-$C_4$)-Alkoxy, ($C_1$-$C_4$)-Alkyl, ($C_1$-$C_4$)-Alkoxycarbonyl, Amino und Trifluormethyl substituiert ist,

($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, Hydroxy, ($C_1$-$C_4$)-Alkoxy, ($C_1$-$C_4$)-Alkyl, ($C_1$-$C_4$)-Alkoxycarbonyl, Amino, ($C_1$-$C_4$)-Alkylamino, Di-($C_1$-$C_4$)-alkylamino, Carboxy, Carboxymethoxy, Amino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl, ($C_1$-$C_4$)-Alkoxycarbonylmethoxy, Carbamoyl, Sulfamoyl, ($C_1$-$C_4$)-Alkoxysulfonyl, Sulfo- und Guanidinomethyl substituiert ist, oder für den Rest eines 3-

bis 8-gliedrigen monocyclischen oder 7- bis 13-gliedrigen bicyclischen Heterocyclus mit mindestens 1 C-Atom, 1-4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet und

A einen N-terminal über $-NR^6-$ mit -CO- und C-terminal über -CO- mit B verknüpften Rest einer natürlichen oder unnatürlichen Aminosäure aus der Reihe Phenylalanin, Histidin, Tyrosin, Tryptophan, β-2-Thienylalanin, β-3-Thienylalanin, β-2-Furylalanin, β-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 2-(2-Thienyl)-serin, (Z)-Dehydrophenylalanin oder (E)-Dehydrophenylalanin, und $R^6$ Wasserstoff bedeutet,

B einen N-terminal über $-NR^7-$ mit A und C-terminal über -CO- mit

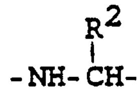

verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]-thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin oder (E)-Dehydrophenylalanin, (1,3-Dioxolan-2-yl)-alanin, N-Pyrrolylalanin, (1-, 3-oder 4-Pyrazolyl)-alanin, (4-Thiazolyl)alanin, (2-, 4-oder 5-Pyrimidyl)-alanin, Cyclopentylglycin, tert.Butylglycin oder Phenylserin und $R^7$ Wasserstoff bedeutet, und

$R^2$ Wasserstoff, $(C_1-C_5)$-Alkyl, $(C_5-C_6)$-Cycloalkyl-$(C_1-C_4)$-alkyl, $(C_6-C_{10})$-Aryl-$(C_1-C_4)$-alkyl oder (Heterocyclyl)-$(C_1-C_4)$-alkyl bedeutet, wobei der Heterocyclus 5 oder 6 Ringglieder, davon 1 oder 2 Schwefel- und/oder Sauerstoffatome, besitzt,

$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,

X wahlweise abwesend sein kann oder für -O-, -S-, $-CF_2-$, -CO- oder $-CHR^8-$ steht, worin

$R^8$ Wasserstoff, $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,

$R^4$ -OH, $-NH_2$ oder 5- oder 6-gliedriges Heteroaryl, das 1 oder 2 Stickstoffatome enthält, das teilweise oder vollständig hydriert sein kann, und das gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet,

p und q unabhängig voneinander 0, 1, 2, 3 oder 4 bedeuten,

$R^5$ die gleiche Bedeutung wie $R^1$ besitzt oder aber zusammen mit $R^1$ und dem sie verbindenden Stickstoffatom einen drei- bis achtgliedrigen Monocyclus oder einen sieben-bis dreizehngliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet.

3. Verfahren gemäß Anspruch 1 und/oder 2, dadurch gekennzeichnet, daß

$R^1$ Wasserstoff, $(C_1-C_{10})$-Alkyl, das gegebenenfalls bis zu drei gleiche oder verschiedene Reste aus der Reihe Hydroxy, Carboxy, Amino, $(C_1-C_4)$-Alkylamino, Di-$(C_1-C_4)$-alkylamino oder $(C_1-C_4)$-Alkoxycarbonylamino substituiert ist,

Phenyl, das gegebenenfalls durch einen oder zwei Reste aus der Reihe F, Cl, Hydroxy, Amino oder Trifluormethyl substituiert ist,

Phenyl-$(C_1-C_4)$-alkyl, worin der Phenylteil gegebenenfalls durch einen oder zwei gleiche oder verschiedene

Reste aus der Reihe F, Cl, Hydroxy, Amino, ($C_1$-$C_4$)-Alkylamino, Di-($C_1$-$C_4$)-alkylamino, Carboxy, Amino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl substituiert ist, bedeutet,
A einen N-terminal über -$NR^6$- mit -CO- und C-terminal über -CO- mit B verknüpften Rest einer Aminosäure aus der Reihe Phenylalanin, Tyrosin, β-2-Thienylalanin, β-3-Thienylalanin, 4-Chlorphenylalanin, O-Methyltyrosin, 1-Naphthylalanin, 2-Naphthylalanin, 2-Fluorphenylalanin, 3-Fluorphenylalanin, 4-Fluorphenylalanin und $R^6$ Wasserstoff bedeutet,
B einen N-terminal über -$NR^7$- mit A und C-terminal über -CO- mit -NH-$CHR^2$- verknüppften Rest einer Aminosäure aus der Reihe Phenylalanin, Histidin, Leucin, β-2-Thienylalanin, β-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)-alanin, (1-, 3- oder 4-Pyrazolyl)-alanin, 4-Thiazolylalanin, (2-, 4- oder 5-Pyrimidyl)-alanin und $R^7$ Wasserstoff bedeutet,
$R^2$ Isobutyl, Cyclohexylmethyl, Benzyl oder (1,3-Dithiolan-2-yl)-methyl bedeutet,
$R^3$ Wasserstoff bedeutet,
X wahlweise abwesend sein kann oder für -S-, -CO- oder -$CHR^8$- steht, worin
$R^8$ Wasserstoff, Hydroxy, ($C_1$-$C_5$)-Alkoxy oder Fluor bedeutet,
$R^4$ 2-Imidazolyl, 4-Imidazolyl, 5-Imidazolyl, 2-Pyridyl, 3-Pyridyl, 4-Pyridyl, 2-Piperidyl, 3-Piperidyl, 4-Piperidyl, 1-Piperazinyl oder 2-Piperazinyl bedeutet, wobei die genannten heterocyclischen Reste jeweils mit ein oder zwei gleichen oder verschiedenen Resten aus der Reihe Fluor, Methoxy, Methyl, Ethyl, ($C_1$-$C_4$)-Alkoxycarbonyl, Amino oder Trifluormethyl substituiert sein können,
p und q unabhängig voneinander 0, 1 oder 2 bedeuten,
$R^5$ die gleiche Bedeutung wie $R^1$ besitzt oder aber zusammen mit $R^1$ und dem sie verbindenden Stickstoffatom einen Rest aus der Reihe Pyrrolidinyl, Piperidyl, Morpholinyl, Thiomorpholinyl, Piperazinyl, Homopiperazinyl oder Hexahydroazepinyl bedeutet, wobei die genannten Reste jeweils mit ein oder zwei gleichen oder verschiedenen Substituenten aus der Reihe Methyl, Ethyl, Propyl, Isopropyl, n-Butyl, s-Butyl, Isobutyl, tert.Butyl, Hydroxy, Hydroxymethyl, Hydroxyethyl, Methoxy, Carboxy, Carboxymethyl, Carboxyethyl, Methoxycarbonyl, Ethoxycarbonyl, ($C_1$-$C_2$)-Alkoxycarbonyl-($C_1$-$C_4$)-alkyl, Fluor, Chlor, Brom, Amino, Amino-($C_1$-$C_4$)-alkyl, ($C_1$-$C_4$)-Alkylamino-, ($C_1$-$C_4$)-Alkylamino-($C_1$-$C_4$)-alkyl, Di-($C_1$-$C_4$)-alkylamino oder Di-($C_1$-$C_4$)-alkylamino-($C_1$-$C_4$)-alkyl substituiert sein können.

4. Verwendung einer Verbindung gemäß einem der Ansprüche 1 bis 3 als Heilmittel.

5. Verwendung einer Verbindung gemäß einem der Ansprüche 1 bis 3 als Heilmittel bei der Behandlung des Bluthochdrucks.

6. Verwendung einer Verbindung gemäß einem der Ansprüche 1 bis 3 als Heilmittel bei der Behandlung von Viruserkrankungen.

7. Verfahren zur Herstellung einer pharmazeutischen Zubereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.